# EUROPÄISCHE PATENTSCHRIFT

(11) **EP 1 984 045 B1**
(45) Veröffentlichungstag und Bekanntmachung des Hinweises auf die Patenterteilung: **18.04.2012**
(21) Anmeldenummer: 08715681.6
(22) Anmeldetag: 01.02.2008
(51) Int. Cl.: A61M 5/178, G21F 5/018

(54) **VORRICHTUNG ZUM BEFÜLLEN EINES MEDIZINISCHEN GERÄTS MIT EINER RADIOAKTIVEN SUBSTANZ UND VERFAHREN**
APPARATUS AND METHOD FOR FILLING A MEDICAL INSTRUMENT WITH A RADIOACTIVE SUBSTANCE
DISPOSITIF POUR REMPLIR UN APPAREIL MÉDICAL AVEC UNE SUBSTANCE RADIOACTIVE ET PROCÉDÉ CORRESPONDANT

(30) Priorität: 07.02.2007 DE 102007006189
(43) Veröffentlichungstag der Anmeldung: 29.10.2008
(73) Patentinhaber: Isotopen Technologien München AG, 83435 Bad Reichenhall (DE)
(72) Erfinder: SCHILP, Michael, 93047 Regensburg (DE); TUOMO, Nikula, 85521 Ottobrunn (DE); BUCK, Oliver, 83457 Bayerisch Gmain (DE)
(74) Vertreter: Hartig, Michael
(86) Internationale Anmeldenummer: PCT/EP2008/000825
(87) Internationale Veröffentlichungsnummer: WO 2008/095655

(56) Entgegenhaltungen:
- WO-A-99/42165
- WO-A-2006/108026
- US-A- 4 208 588
- US-A- 4 241 728
- US-A- 4 401 108
- US-A- 5 961 439
- US-A- 6 162 198
- US-A1- 2004 015 038

## Beschreibung

Die vorliegende Erfindung betrifft eine Vorrichtung zum Befüllen eines medizinischen Instruments mit einer radioaktiven Substanz und ein entsprechendes Verfahren.

Auf dem Gebiet der Nuklearmedizin werden radioaktive Substanzen für verschiedene diagnostische und therapeutische Zwecke eingesetzt. Ein Beispiel ist die Angioplastie, bei der verengte oder verschlossene Blutgefäße in einem menschlichen oder tierischen Körper mit Hilfe einer Ballondilatation erweitert oder wieder geöffnet werden. Nach dem Einführen eines Ballonkatheters in ein Blutgefäß und einem Aufweiten oder Wiedereröffnen des Blutgefäßes besteht ein hohes Risiko einer Narbenbildung (Restenose) durch die bei der Erweiterung des Blutgefäßes erfolgende mikroskopische Verletzung der Innenwand des Blutgefäßes. Zur Verhinderung der Narbenbildung kann ein mit einem Medikament versehener Stent an der erweiterten Stelle des Butgefäßes angeordnet werden, von dem kontinuierlich Zellwachstumsinhibitoren abgegeben werden, wodurch die Restenose verhindert wird. Eine vielversprechende Alternative ist das Befüllen eines Dilatationsballons mit einer radioaktiven Flüssigkeit, wobei die Gefäßwände durch die von dieser abgegebene radioaktive Strahlung lokal verödet werden und eine Narbenbildung ebenfalls verhindert werden kann. Durch die Anwendung radioaktiver Strahlung wird das Risiko einer Restenose um ein Vielfaches gegenüber einem erweiterten oder wieder geöffneten jedoch danach unbehandelten Blutgefäß gesenkt, ohne daß der Körper des Patienten durch ein Medikament mit Giftstoffen belastet wird. Die Anwendung radioaktiver Strahlung stellt jedoch besondere Anforderungen und Vorkehrungen an das Klinikumfeld. Unter anderem ist das Befüllen des Katheters mit radioaktiven Flüssigkeiten für den Patienten und das medizinische Personal mit einem erhöhten Risiko verbunden.

Üblicherweise werden für eine radioaktive Behandlung verwendete Katheter manuell mit Hilfe einer Spritze befüllt. Eine Behandlung umfaßt in der Regel das Befüllen der Spritze mit einer flüssigen radioaktiven Substanz aus einem Behälter, was meist in einem Labor geschieht, den Transport der dann mit der radioaktiven Flüssigkeit gefüllten Spritze zum Patienten, das Legen eines Katheters durch den Arzt, das Anschließen des Katheters an die Spritze und Befüllen desselben, das Entleeren des Katheters und das Entfernen des Katheters aus dem Patienten. Nach dem Befüllen der Spritze werden der Arzt sowie klinisches Personal und auch der Patient durch von der radioaktiven Flüssigkeit abgegebene radioaktive Strahlung belastet. Beim Patienten erfolgt die Strahlenbelastung zusätzlich vor und nach der eigentlichen Behandlung des Blutgefäßes beim Befüllen und Entleeren des Katheters und auch bereits beim Transport der mit der radioaktiven Flüssigkeit gefüllten Spritze vom Labor zum Patienten.

Eine aus dem Stand der Technik bekannte Vorrichtung zum Befüllen eines medizinischen Instruments mit einer radioaktiven Substanz ist im Dokument US-A-4 401 108 offenbart.

Es ist die Aufgabe der vorliegenden Erfindung eine Vorrichtung und ein Verfahren zum Befüllen eines medizinischen Instruments, wie beispielsweise eines Katheters bereitzustellen, mit der bzw. dem die Strahlenbelastung von Patienten und medizinischem Personal, insbesondere beim Befüllen des medizinischen Instruments reduziert werden kann.

Diese Aufgabe wird gelöst durch eine Vorrichtung mit den Merkmalen des Anspruchs 1 sowie ein Verfahren mit den Merkmalen des Anspruchs 17.

Vorteilhafte Ausführungsformen sind in den Unteransprüchen angegeben.

Erfindungsgemäß wird eine Vorrichtung zum Befüllen eines medizinischen Instruments mit einer radioaktiven Substanz bereitgestellt, welche Vorrichtung ein zur Aufnahme einer Spritze und zumindest eines Teils des medizinischen Instruments eingerichtetes, radioaktive Strahlung abschirmendes Gehäuse und eine Einrichtung zur Aufnahme eines Behälters für eine radioaktive Substanz umfaßt.

Mit Hilfe der erfindungsgemäßen Vorrichtung kann eine wesentliche Reduzierung der Strahlenbelastung durch eine radioaktive Substanz, insbesondere radioaktive Flüssigkeit beim Befüllen eines medizinischen Instruments, wie beispielsweise eines Katheters, insbesondere eines Ballonkatheters erreicht werden, da die Vorrichtung so eingerichtet ist, daß das Befüllen und Entleeren medizinischen Instruments in einem Strahlung abschirmenden Gehäuse erfolgt. Mit der erfindungsgemäßen Vorrichtung können eine zum Befüllen eines medizinischen Instruments verwendete Spritze und zumindest ein Teil des medizinischen Instruments sowie ein Behälter, in dem eine zu verabreichende radioaktive Substanz enthalten ist, in einer gegen radioaktive Strahlung abgeschirmten Weise miteinander verbunden werden, so daß das eine Behandlung durchführende oder vorbereitende medizinische Personal und auch der Patient selbst beim Befüllen und Entleeren des Katheters vor radioaktiver Strahlung wesentlich geschützt sind. Da vor dem Befüllen des Katheters die Spritze, der Katheter und der Behälter für die radioaktive Substanz miteinander verbunden und abgeschirmt werden können, wird der Transport einer mit einer radioaktiven Flüssigkeit befüllten Spritze ohne Abschirmung vermieden.

Gemäß einer bevorzugten Ausführungsform ist die Einrichtung zur. Aufnahme des Behälters für eine Anordnung und Befestigung des Behälters außerhalb des Gehäuses eingerichtet und umfaßt das Gehäuse eine Öffnung zur Herstellung einer Verbindung mit dem Behälter zur Zufuhr einer radioaktiven Substanz in das Gehäuse. Dadurch können Behälter zur Aufbewahrung radioaktiver Substanzen unabhängig von ihrer Größe mit der Vorrichtung verbunden und des weiteren die Vorrichtung in platzsparender Weise konzipiert werden, da der Behälter nicht innerhalb des Gehäuses der Vorrichtung aufgenommen werden muß. Da der Behälter vorzugsweise zum Transport und zur Lagerung einer radioaktiven Substanz ohnehin eine eigene radioaktive Abschirmung aufweist bzw. aus einem entsprechenden Material hergestellt ist, bietet eine Aufnahme des Behälters im abgeschirmten Gehäuse selbst keine Vorteile. Jedoch ist auch eine derartige Ausführungsform der Vorrichtung denkbar. Da die Vorrichtung und der Behälter als separate Einheiten vorgesehen werden können, ist eines einfaches und risikofreies Handhaben und Abfüllen der radioaktiven Substanz beispielsweise in einem Labor mit Hilfe geeigneter Vorrichtungen und durch Fachpersonal möglich, ohne daß Ärzte oder medizinisches Personal damit belastet werden.

Gemäß einer weiteren bevorzugten Ausführungsform weist das Gehäuse einen Deckel auf, wobei der Deckel dazu eingerichtet ist, die Vorrichtung und einen am Gehäuse angeordneten Behälter für eine radioaktive Substanz abzudecken oder zu verschließen. Bei geöffnetem Deckel können Komponenten, wie beispielsweise eine Spritze, ein medizinisches Instrument, Ventile und Schlauchabschnitte zur Verbindung in das Gehäuse eingelegt und mit einander Verbunden werden, während eine radioaktive Substanz vorzugsweise erst bei geschlossenem Deckel in die Spritze bzw. das medizinische Instrument zugeführt wird.

Da der Deckel auch den Behälter für die radioaktive Substanz abdecken bzw. verschließen kann, kann der Behälter beispielsweise so ausgelegt sein, daß nach dem Anordnen desselben an der Vorrichtung ein Deckel des Behälters abgenommen wird und mit Hilfe eines oder mehrere Schläuche eine Verbindung zwischen dem Behälter, der Spritze und dem medizinischen Instrument hergestellt wird, wobei danach alle Komponenten einschließlich des Behälters durch Schließen des Deckels gegen radioaktive Strahlung abgeschirmt werden.

Es ist jedoch auch denkbar, einen Deckel vorzusehen, der lediglich das Gehäuse der Vorrichtung, jedoch nicht den Behälter verschließt. Bei dieser Ausführungsform könnte der Behälter mit einer gegen radioaktive Strahlung geschützten Anschlußleitung zur Verbindung mit dem Gehäuse versehen sein.

Gemäß einer weiteren bevorzugten Ausführungsform umfaßt das Gehäuse eine von außen betätigbare Einrichtung, wie beispielsweise einen durch die Gehäusewand geführten Drehhebel, der zur Bedienung eines im Gehäuse angeordneten Ventils mit diesem in Eingriff gebracht werden kann. Bei dem Ventil kann es sich beispielsweise um ein im medizinischen Bereich häufig verwendetes Kunststoffventil zur Verbindung von Schläuchen, insbesondere ein Drei- oder Mehrwegeventil handeln, das in dem Gehäuse angeordnet werden kann und von außen durch den Drehhebel bedient werden kann.

Gemäß einer weiteren bevorzugten Ausführungsform ist das Gehäuse aus einem radioaktive Strahlung abschirmenden Material, insbesondere Wolfram, hergestellt [was sind die besonderen Vorzüge von Wolfram hinsichtlich der erfindungsgemäßen Vorrichtung? Welche Materialien eignen sich ebenfalls]. Es können jedoch auch andere zur Strahlungsabschirmung geeignete Materialien verwendet werden.

Gemäß noch einer weiteren Ausführungsform kann das Gehäuse eine Fensteröffnung aufweisen. In der Fensteröffnung kann ein Bleifenster angeordnet sein. Damit kann der Vorgang des Befüllens einer Spritze bei geschlossenem Deckel beobachtet werden, um beispielsweise eine Blasenfreiheit der aufgezogenen Flüssigkeit sicherzustellen. Alternativ oder zusätzlich kann das Gehäuse auch eine Klappe umfassen, mit der das Fenster verschlossen werden kann. An der Innenseite der Klappe kann zusätzlich ein Spiegel angeordnet sein, um den Füllvorgang bei teilweise geöffneter Klappe zu beobachten. Die Gewährleistung der Blasenfreiheit der radioaktiven Flüssigkeit ist von hoher Bedeutung, da Luft im Katheterschlauch unter Umständen ein vollständiges Befüllen des Katheters verhindern kann, so daß der Ballon teilweise oder ganz mit Luft befüllt ist und die radioaktive Flüssigkeit im Schlauch verbleibt und eine Behandlung des Blutgefäßes an der gewünschten Stelle unterbleibt.

Gemäß noch einer weiteren Ausführungsform ist das Gehäuse zur Fixierung der Spritze und des Teils des medizinischen Instruments eingerichtet und auch zur Aufnahme und Fixierung eines oder mehrerer Ventile und Verbindungsleitungen im Gehäuse. Die Aufnahme und Fixierung der Komponenten kann beispielsweise dadurch erfolgen, daß im Inneren des Gehäuses zu den Komponenten oder Teilen, die im Gehäuse angeordnet werden sollen, formschlüssige Haltevorrichtungen vorgesehen werden. Die Haltevorrichtungen können als Einsatz für das Gehäuse ausgebildet sein oder auch einstückig mit dem Gehäuse hergestellt sein. Es ist auch denkbar, Teile der Haltevorrichtungen sowohl im Deckel als auch im übrigen Gehäuse vorzusehen, so daß nach einem Einlegen der Komponente(n) in das Gehäuse eine vollständige Fixierung durch das Schließen des Deckels erfolgt.

Gemäß noch einer weiteren Ausführungsform umfaßt das Gehäuse eine Öffnung zur Betätigung eines Spritzenkolbens einer in der Vorrichtung angeordneten Spritze. Dadurch kann die in der Vorrichtung angeordnete Spritze manuell oder auch mit einem Motor von der Außenseite des Gehäuses bedient werden.

Des weiteren kann eine Abschirmungsvorrichtung für das aus der Spritze ragende Ende des Spritzenkolbens vorgesehen werden. Eine Abschirmungsvorrichtung kann beispielsweise so ausgebildet sein, daß sie mit dem Ende des Spritzenkolbens verbunden werden kann und beweglich in einer Öffnung des Gehäuses angeordnet ist oder auf den aus dem Gehäuse ragenden Spritzenkolben aufgesteckt oder auf andere Weise damit verbunden wird. Des weiteren kann die Abschirmungsvorrichtung für den Spritzenkolben auch als eine Verlängerung des Spritzenkolbens ausgebildet sein. Vorzugsweise ist die Abschirmungsvorrichtung für den Spritzenkolben aus demselben Material wie das Gehäuse der Vorrichtung bzw. die Vorrichtung selbst hergestellt. Sie könnte jedoch auch lediglich als Verlängerung des Spritzenkolbens ohne besondere Abschirmungseigenschaften hergestellt sein. Des weiteren kann das Gehäuse der Vorrichtung eine Öffnung für den Durchtritt eines medizinischen Instruments oder Teilen desselben umfassen.

Schließlich kann die Vorrichtung gemäß einer weiteren bevorzugten Ausführungsform einen Ständer umfassen, der so mit der Vorrichtung verbunden ist, daß die Vorrichtung und eine darin angeordnete Spritze im Verhältnis zu einer Standoberfläche, wie beispielsweise einer Tischplatte geneigt sind. Vorzugsweise wird die Neigung der Vorrichtung so vorgesehen, daß eine in der Vorrichtung angeordnete Spritze abwärts geneigt ist, so daß Luftblasen zum Ende der Spritze aufsteigen und dadurch nicht in den Katheter gelangen können.

Gemäß einer weiteren bevorzugten Ausführungsform ist das medizinische Instrument ein Ballonkatheter.

Erfindungsgemäß wird des weiteren ein System zum Befüllen eines Behälters mit einer radioaktiven Substanz bereitgestellt, welches eine erfindungsgemäße Vorrichtung, einen Behälter für eine radioaktive Substanz und eine Spritze umfaßt.

Gemäß einer Ausführungsform kann das System auch ein in der Vorrichtung angeordnetes Ventil, ein zumindest teilweise in der Vorrichtung angeordnetes medizinisches Instrument, wie beispielsweise einen Katheter, und Schlauchabschnitte zur Verbindung der Spritze, des Ventils, des Behälters für eine radioaktive Substanz und des medizinischen Instruments umfassen. In einem derartigen System können eine Spritze, ein Behälter für eine radioaktive Substanz und ein medizinisches Instrument mit Schlauchabschnitten und Ventilen derart verbunden werden, daß durch Umschalten eines Ventils, insbesondere eines Dreiwegeventils, zunächst die Spritze mit der radioaktiven Flüssigkeit und anschließend durch Entleeren der Spritze ein medizinisches Instrument befüllt werden kann. Dabei sind die Spritze, der Behälter für die radioaktive Substanz, der bzw. die Schlauchabschnitte und das bzw. die Ventile und zumindest ein Teil des medizinischen Instruments gegenüber der Umgebung abgeschirmt, wodurch die Strahlenbelastung durch radioaktive Strahlung von Personen in der Umgebung stark reduziert werden kann.

Gemäß einer weiteren bevorzugten Ausführungsform ist der Behälter für eine radioaktive Substanz abnehmbar an der Vorrichtung befestigt. Der Behälter kann dadurch leicht ausgetauscht werden und bildet mit der Vorrichtung dennoch eine stabile Einheit, so daß die Vorrichtung zusammen mit dem Behälter beispielsweise mit einer Hand verschoben oder transportiert werden kann. Des weiteren kann die Vorrichtung mit Behältern unterschiedlicher Größe verbunden werden.

Erfindungsgemäß wird des weiteren ein Verfahren zum Abfüllen einer radioaktiven Substanz bereitgestellt, das ein Einsetzen einer Spritze in eine erfindungsgemäße Vorrichtung, ein Anordnen eines Behälters mit einer radioaktiven Substanz an der Vorrichtung und das Aufziehen einer radioaktiven Substanz in die Spritze umfaßt. Gemäß einer bevorzugten Ausführungsform kann das Verfahren das Anordnen eines medizinischen Instruments, wie beispielsweise eines Katheters, oder eines Teils davon in der Vorrichtung und das Befüllen des Katheters mit einer radioaktiven Substanz aus der Spritze umfassen. Durch das Anordnen der Spritze und eines Teils bzw. des ganzen medizinischen Instruments sowie von Schlauchabschnitten zur Verbindung von Spritze und medizinischem Instrument mit einem Behälter für eine radioaktive Substanz sowie von dazwischen angeordneten Ventilen können Personen, die das Befüllen des medizinischen Instruments vornehmen, sowie damit zu behandelnde Patienten vor radioaktiver Strahlung besser geschützt werden. Dadurch kann die pro Behandlung von den behandelnden Personen aufgenommene Strahlendosis reduziert werden, wodurch einerseits die Gesundheitsgefährdung dieser Personen vermindert wird und andererseits die zulässige Anzahl der von den Personen durchgeführten Behandlungen, aufgrund der geringeren Strahlenbelastung erhöht werden kann. Für eine weitere Reduzierung der Strahlenbelastung kann das Verfahren ein Aufziehen der Spritze unter Verwendung einer Abschirmungsvorrichtung für das aus der Spritze ragende Ende des Spritzenkolbens umfassen.

Im Folgenden wird die Erfindung mit Hilfe der beigefügten Zeichnung anhand einer beispielhaften Ausführungsform beschrieben, wobei in der Zeichnung:
- Fig. 1: die erfindungsgemäße Vorrichtung in geöffneter Position in einer perspektivischen Ansicht von oben zeigt;
- Fig. 2: die erfindungsgemäße Vorrichtung gemäß der in Fig. 1 gezeigten Ausführungsform in geschlossener Position in einer perspektivischen Ansicht von oben zeigt;
- Fig. 3: die erfindungsgemäße Vorrichtung wie in Fig. 2 jedoch mit geöffneter Klappe zeigt; und

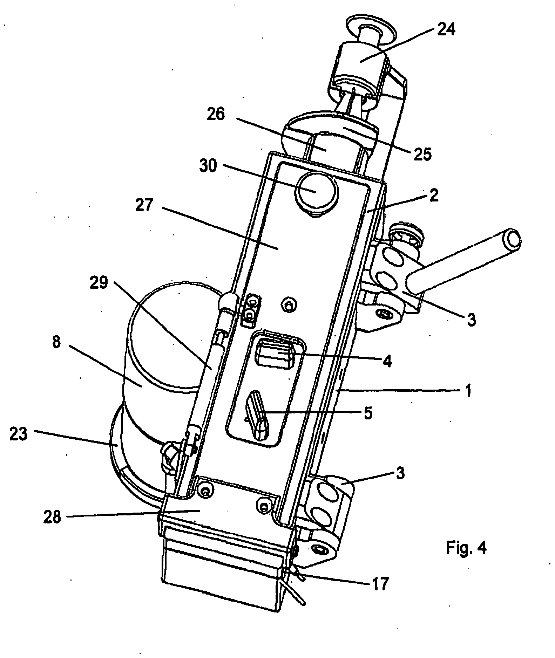

- Fig. 4: die erfindungsgemäße Vorrichtung gemäß der in Fig. 1 gezeigten Ausführungsform in geschlossener Position in einer anderen perspektivischen seitlichen Ansicht zeigt.

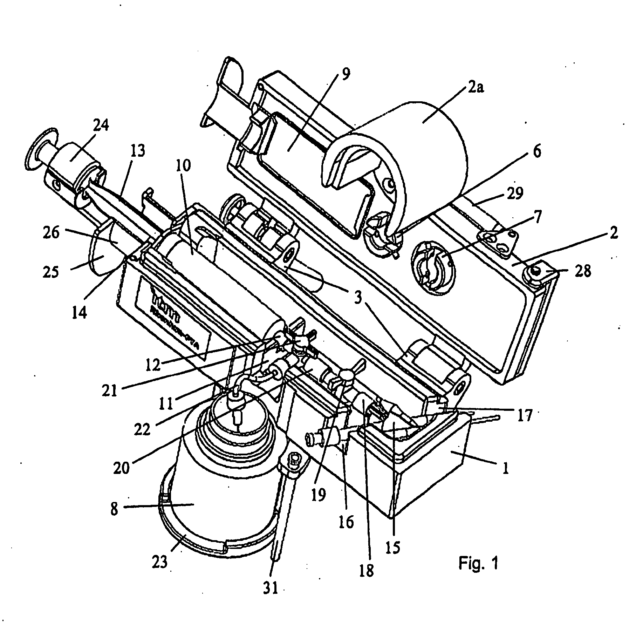

Die erfindungsgemäße Vorrichtung wird im Folgenden anhand der in den Figuren dargestellten beispielhaften Ausführungsform beschrieben. Wie insbesondere in Fig. 1 zu erkennen ist, umfaßt die Vorrichtung ein Gehäuse 1 und einen Deckel 2, die mit Hilfe von Scharnieren 3 miteinander verbunden sind. Zum Verschließen des Gehäuses kann der Deckel 2 auf das Gehäuse geschwenkt werden. Des weiteren sind am Deckel 2 Drehhebel 4, 5 angeordnet, die durch den Deckel geführt sind und im Inneren jeweils eine Mitnahmevorrichtung 6 bzw. 7 für Ventilhebel aufweisen. Drehhebel könnten zusätzlich oder alternativ auch in der Gehäusewand vorgesehen sein.

Der Deckel 2 weist des weiteren einen Abschnitt 2a auf, der im auf das Gehäuse 1 geklappten Zustand über das Gehäuse 1 zur Seite hinausragt und zur Abdeckung bzw. zum Verschließen eines zur Aufnahme von radioaktiver Flüssigkeit geeigneten Behälters 8 vorgesehen ist. Im Deckel 2 ist eine Fensteröffnung 9 vorgesehen. In der Fensteröffnung 9 kann eine Scheibe (nicht gezeigt) angeordnet werden, die beispielsweise zur Abschirmung radioaktiver Strahlung aus Bleiglas hergestellt sein kann. Wie in Fig. 1 zu erkennen ist, ist im Inneren eines Gehäuses 1 eine Spritze 10 angeordnet, die aus dem Gehäuse 1 entnommen werden kann. Bei der Spritze 10 kann es sich um jede herkömmliche Spritze, wie beispielsweise eine Einweg-Kunststoffspritze oder eine Mehrweg-Glasspritze handeln. Im Gehäuse 1 ist des weiteren senkrecht zur Längsrichtung eine stegförmig ausgebildete Aufnahmevorrichtung 11 für die Spritze 10 vorgesehen, die eine zur formschlüssigen Aufnahme der Düse der Spritze 10 geeignete U-förmige Vertiefung aufweist. Die Aufnahmevorrichtung 11 ist so in einer Entfernung von einer stirnseitigen Wand des Gehäuses 1 vorgesehen, daß die Spritze 10 zwischen der Aufnahmevorrichtung 11 und der stirnseitigen Wand des Gehäuses 1 eingesetzt werden kann, wobei ein Spritzenkolben 13 der Spritze 10 durch eine in der Wand des Gehäuses 1 ausgebildete Öffnung 14 nach außen ragt. Wie in Fig. 1 zu erkennen ist, ist der Abstand zwischen der Aufnahmevorrichtung 11 und der stirnseitigen Öffnung des Gehäuses der Länge des Spritzenkörpers entsprechend gewählt, so daß das Ende des Spritzenkörpers bei eingesetzter Spritze 10 gegen die stirnseitige Gehäusewand anliegt.

In Fig. 1 ist ferner ein Teil eines medizinischen Instruments 15, wie beispielsweise eines Ballonkatheters zu erkennen, der im Gehäuse in der Nähe der Gehäusewand angeordnet ist, die der stirnseitigen Gehäusewand mit der für den Spritzenkolben 13 vorgesehenen Öffnung 14 gegenüberliegt. Um das eine T-förmige Verzweigung aufweisende Teil des medizinischen Instruments 15 im Gehäuse 1 anordnen zu können, sind zwei weitere Öffnungen 16, 17 für den Durchtritt des medizinischen Instruments 15 in der Gehäusewand vorgesehen. Das im Inneren des Gehäuses 1 angeordnete Ende der Verzweigung des medizinischen Instruments 15 ist mit Hilfe eines Schlauchabschnitts 18 mit einem Ventil 19 verbunden, das beim Schließen des Deckels 2 mit der entsprechend ausgebildeten Mitnahmevorrichtung 7 des Drehhebels 5 in Eingriff gebracht wird. Das Ventil 19 ist über einen weiteren Schlauchabschnitt 20 mit einem Dreiwege-Ventil 21 verbunden, das bei geschlossenem Deckel 2 mit der Mitnahmevorrichtung 6 des Drehhebels 4 in Eingriff gebracht wird. Das Dreiwege-Ventil 21 ist mit einem weiteren Schlauchabschnitt 22 mit dem Behälter 8 für eine radioaktive Substanz verbunden, der in einer Aufnahmevorrichtung 23 am Gehäuse 1 angeordnet ist. Der Behälter 8 kann aus einer Aufnahme 23 am Gehäuse 1 entnommen werden. Die Verbindung zwischen dem medizinischen Instrument 15, den Ventilen 19, 21, den Schlauchabschnitten 18, 20, 22 und der Spritze 10 erfolgt vorzugsweise über Luer-Lock-Verbindungen oder Steckverbindungen.

In Fig. 1 ist des weiteren eine Abschirmungsvorrichtung 24 für das aus der Spritze 10 ragende Ende des Spritzenkolbens 13 dargestellt. Die Abschirmungsvorrichtung 24 wird mit dem Ende des Spritzenkolbens 13 verbunden und ist so ausgebildet, daß sie auf das Scheibenförmige Ende des Spritzenkolbens 13 von der Seite aufgeschoben werden kann. Die Abschirmungsvorrichtung 24 ist vorzugsweise aus demselben Material wie das Gehäuse 1 der Vorrichtung, beispielsweise aus Wolfram, hergestellt. Sie kann jedoch auch aus einem anderen Material hergestellt sein. Wie in Fig. 1 zu erkennen ist, bildet die Abschirmungsvorrichtung 24 eine Verlängerung des Spritzenkolbens 13, um den Finger einer den Spritzenkolben 13 betätigenden Person weiter von der in der Spritze 10 aufgenommenen radioaktiven Flüssigkeit zu entfernen. Auch das Material der Abschirmungsvorrichtung 24 dient zusätzlich der Strahlungsabschirmung.

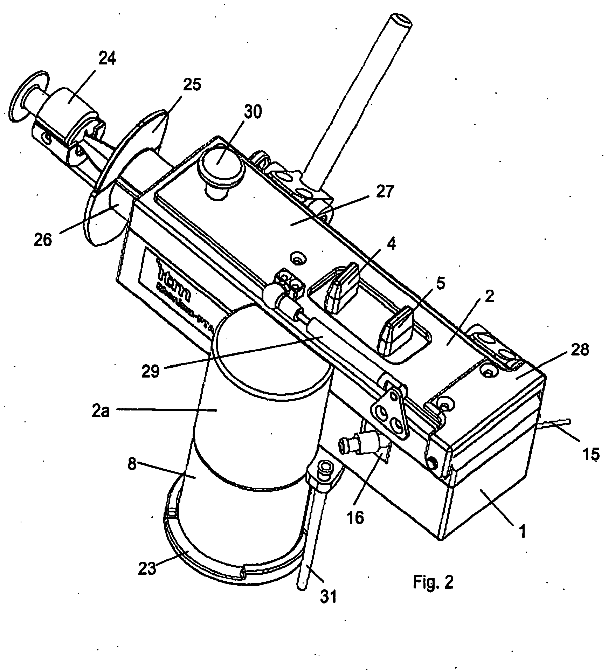

Die Vorrichtung umfaßt des weiteren eine ringförmige Haltefläche 25, die am Ende eines rohrförmigen, das Gehäuse fortsetzenden Abschnitts 26 angeordnet ist, der den Spritzenkolben 13 umgibt. Die senkrecht zum rohrförmigen Abschnitt 26 angeordnete Haltefläche 25 dient dazu, daß die in das Gehäuse 1 eingelegte Spritze 10 mit den Fingern einer einzigen Hand betätigt werden kann. Wie in Fig. 1 und 2 zu erkennen ist, setzen sich die Haltefläche 25 und der den Spritzenkolben 13 umgebende rohrförmige Abschnitt 26 aus zwei Teilen zusammen, die am Gehäuse und am Deckel 2 angeordnet sind, die sich nach dem Schließen des Deckels 2 zu einem Teil ergänzen.

In Fig. 2 ist der mit der Abschirmungsvörrichtung 24 versehene Spritzenkolben 13 im wesentlichen vollständig aus der Spritze 10 herausgezogen gezeigt. Wird der Spritzenkolben 13 in die Spritze gedrückt, tritt die Abschirmungsvorrichtung 24 in den rohrförmige Abschnitt 26 ein und wird in diesem aufgenommen.

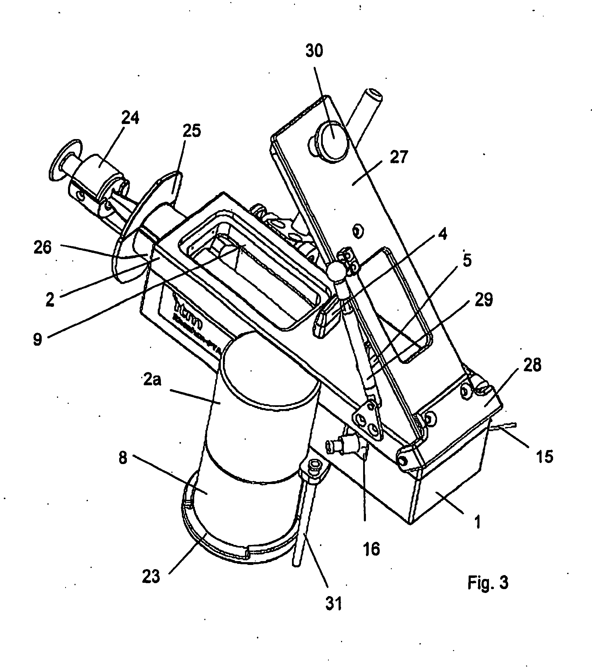

Am Deckel 2 ist des weiteren eine Kappe 27 zum Verschließen der Fensteröffnung 9 angeordnet, die mit Hilfe eines Scharniers 28 am Deckel 2 schwenkbar befestigt ist. Die Klappe 27 umfaßt eine Metallplatte, die auf die dem Gehäuse 1 gegenüberliegende Oberfläche des Dekkels 2 geklappt werden kann. Die Metallplatte weist eine der Form der Fensteröffnung 9 entsprechende Verdickung auf, die die Fensteröffnung 9 bei auf den Deckel 2 abgesenkter Klappe 27 ausfüllt. Die Klappe 27 umfaßt des weiteren eine Öffnung 28 für den Durchgang der Drehhebel 4 und 5. Um das Schwenken der Klappe zu begrenzen und die Klappe geöffnet halten zu können, wie in Fig. 3 gezeigt ist, wird ein an der Klappe 27 und am Deckel 2 befestigter Teleskoparm 29 vorgesehen. Auf der Klappe 27 ist des weiteren ein Knauf 30 zum Öffnen und Schließen angebracht. Auf der Innenseite der Klappe 27 und insbesondere auf der Verdickung kann ein (nicht gezeigter) Spiegel angeordnet sein, der die Betrachtung der Spritze 10 im Gehäuse 2 bei teilweise geöffneter Klappe 27 erleichtert.

Die Vorrichtung weist eine Länge von ungefähr 20 cm, eine Höhe von ungefähr 3 cm und eine Breite von ungefähr 7 cm auf. Die Wandstärke des Gehäuses beträgt ca. 0,75 mm. Es können jedoch auch geringere oder größere Abmessungen gewählt werden.

Eine Verwendung der Vorrichtung kann in der folgenden Weise erfolgen. Zunächst wird eine Spritze 10 in das Gehäuse 1 und darauffolgend das Ende bzw. Verzweigungsstück eines Katheters 15 in das Gehäuse 1 eingelegt, die mit Hilfe von Schlauchabschnitten 18, 20 über die Ventile 19 und 21 miteinander verbunden werden. Schließlich wird ein Behälter 8 mit einer für eine Behandlung vorgesehenen radioaktiven Flüssigkeit in die Aufnahme 15 der Vorrichtung eingesetzt und am Dreiwege-Ventil 21 mit der Spritze 10 und dem Katheter 15 verbunden. Das Gehäuse 1 wird darauffolgend mit dem Deckel 2 verschlossen, wobei die Mitnahmeeinrichtungen 6 und 7 der Drehhebel 4 und 5 der Vorrichtung mit den Hebeln der Ventile 19 und 21 in Eingriff gebracht werden. Die Vorrichtung ist jetzt für eine Verwendung vorbereitet.

In einem ersten Anwendungsschritt wird die Spritze 10 mit der im Behälter 8 enthaltenen Flüssigkeit befüllt, wobei die Ventile 19, 21 so eingestellt sind, daß der Katheter 15 abgekoppelt ist. Nach dem Befüllen der Spritze 10 wird das Ventil 21 so umgestellt, daß der Behälter 8 abgekoppelt ist. Durch Drücken des mit der Abschirmungsvorrichtung 24 versehenen Spritzenkolbens 13 kann jetzt der Inhalt der Spritze 10 nach Umstellen des Ventils 19 in den Katheter 15 gepreßt werden. Dabei kann das Befüllen der Spritze durch Beobachtung der Spritze durch das geöffnete Fenster 9 und gegebenenfalls unter Verwendung des an der Klappe 27 angeordneten Spiegels überwacht werden. Der gesamte Vorgang des Befüllens des Katheters 15 findet unter Abschirmung durch das Gehäuse 1 bzw. den Deckel 2 und gegebenenfalls unter Verwendung der Abschirmungsvorrichtung 24 für den Spritzenkolben 13 statt, so daß die an sich in der Nähe befindenden Personen abgegebene Strahlung deutlich reduziert wird.

An der beschriebenen Ausführungsform können zahlreiche Abwandlungen vorgenommen werden, ohne den Umfang der vorliegenden Erfindung zu verlassen. Beispielsweise wäre es denkbar, die Spritze 10 vollständig innerhalb des Gehäuses 1 anzuordnen und das Aufziehen bzw. Befüllen und Entleeren der Spritze 10 mit Hilfe eines Motors durchzuführen. Des weiteren könnte der Behälter 8 auch innerhalb des Gehäuses 1 angeordnet bzw. fixiert sein. Ferner ist denkbar, mehrere Aufnahmen für Behälter am oder im Gehäuse vorzusehen.

Des weiteren kann am Gehäuse 1 ein Ständer befestigt sein, der es ermöglicht, die Vorrichtung zusammen mit dem daran angeordneten Behälter 8 auf einer Unterlage aufzustellen. Der Ständer kann über eine Haltevorrichtung 31 so am Gehäuse 1 befestigt sein, daß das Gehäuse im Verhältnis zur Unterlage geneigt ist, so daß beim Befüllen des Katheters Luftblasen in der Spritze zu deren Ende aufsteigen und nicht in den Katheter gelangen können.

Ferner kann das Gehäuse 1 so aufgebaut sein, daß ein medizinisches Instrument oder ein Katheter vollständig darin aufgenommen werden können und keine Öffnungen für den Durchtritt eines Katheters im Gehäuse 1 vorgesehen werden müssen.

Auch bezüglich der Verbindung zwischen dem Behälter 8 und der Spritze 10 und dem Katheter 15 sind andere Realisierungsmöglichkeiten denkbar. Beispielsweise könnte die Kopplung zwischen dem Behälter und dem Gehäuse mit Hilfe einer automatischen Schnittstelle erfolgen. Auch könnte der Deckel 2 ohne den Fortsatz 2a für den Behälter 8 vorgesehen werden, wenn gleichzeitig ein vollständig abgeschirmter Behälter 8 verwendet wird.

Die in der Beschreibung und in den Ansprüchen angegebenen Merkmale können in jeder beliebigen Kombination für die Erfindung von Bedeutung sein.

## Patentansprüche

1. Vorrichtung zum Befüllen eines medizinischen Instruments mit einer radioaktiven Substanz, welche Vorrichtung ein zur Aufnahme einer Spritze (10) und zumindest eines Teils des medizinischen Instruments (15) eingerichtetes, radioaktive Strahlung abschirmendes Gehäuse (1), eine Abschirmungsvorrichtung (24) für ein aus einer Spritze (10) ragendes Ende des Spritzenkolbens (13) und eine Einrichtung (23) zur Aufnahme eines Behälters (8) für eine radioaktive Substanz umfaßt, wobei das Gehäuse (1) einen Deckel (2), der eingerichtet ist, die Vorrichtung und einen am Gehäuse (2) angeordneten Behälter (8) für eine radioaktive Substanz abzudecken oder zu verschließen und eine Öffnung (14) zur Betätigung des Spritzenkolbens (13) der in der Vorrichtung angeordneten Spritze (10) aufweist.

2. Vorrichtung nach Anspruch 1, **dadurch gekennzeichnet, daß** die Einrichtung (23) zur Aufnahme des Behälters (8) für eine Anordnung und Befestigung des Behälters (8) außerhalb des Gehäuses (1) eingerichtet ist und das Gehäuse (1) eine Öffnung zur Herstellung einer Verbindung mit dem Behälter (8) zur Zufuhr einer radioaktiven Substanz in das Gehäuse (1) umfaßt.

3. Vorrichtung nach einem der vorhergehenden Ansprüche, **dadurch gekennzeichnet, daß** das Gehäuse (1) eine von außen betätigbare Einrichtung (4, 5) zur Bedienung eines im Gehäuse (1) angeordneten Ventils (19, 21) umfaßt.

4. Vorrichtung nach einem der vorhergehenden Ansprüche, **dadurch gekennzeichnet, daß** sie aus einem radioaktive Strahlung abschirmenden Material, insbesondere Wolfram, hergestellt ist.

5. Vorrichtung nach einem der vorhergehenden Ansprüche, **dadurch gekennzeichnet, daß** das Gehäuse (1) ein Fenster (9) und eine Klappe (27) umfaßt, mit der das Fenster (9) verschlossen werden kann.

6. Vorrichtung nach einem der vorhergehenden Ansprüche, **dadurch gekennzeichnet, daß** das Gehäuse (1) zur Fixierung der Spritze (10) und des Teils des medizinischen Instruments (15) und zur Aufnahme und Fixierung eines oder mehrerer Ventile (19, 21) und Verbindungsleitungen (18, 20, 22) im Gehäuse (1) eingerichtet ist.

7. Vorrichtung nach einem der vorhergehenden Ansprüche, **dadurch gekennzeichnet, daß** das Gehäuse (1) eine Öffnung (16, 17) für den Durchtritt des medizinischen Instruments (15) umfaßt.

8. Vorrichtung nach einem der vorhergehenden Ansprüche, **dadurch gekennzeichnet, daß** sie einen Ständer umfaßt, der so mit der Vorrichtung verbunden ist, daß die Vorrichtung und eine darin angeordnete Spritze (10) im Verhältnis zu einer Standoberfläche geneigt sind.

9. Vorrichtung nach einem der vorhergehenden Ansprüche, **dadurch gekennzeichnet, daß** das medizinische Instrument (15) ein Katheter ist.

10. System zum Befüllen eines Behälters mit einer radioaktiven Substanz, welches eine Vorrichtung nach einem der vorhergehenden Ansprüche, einen Behälter (8) für eine radioaktive Substanz und eine Spritze (10) umfaßt.

11. System nach Anspruch 10, **dadurch gekennzeichnet, daß** es ein in der Vorrichtung angeordnetes Ventil (19, 21), ein zumindest teilweise in der Vorrichtung angeordnetes medizinisches Instrument (15) und Schlauchabschnitte (18, 20, 22) zur Verbindung der Spritze (10), des Ventils (19, 21), des Behälters (8) für eine radioaktive Substanz und des medizinischen Instruments (15) umfaßt.

12. System nach Anspruch 10 oder 11, **dadurch gekennzeichnet, daß** der Behälter (8) für eine radioaktive Substanz abnehmbar an der Vorrichtung befestigt ist.

13. Verfahren zum Abfüllen einer radioaktiven Substanz, umfassend die folgenden Schritte:
Einsetzen einer Spritze (10) in eine Vorrichtung gemäß einem der Ansprüche 1 bis 10,
Anordnen eines Behälters (8) mit einer radioaktiven Substanz an der Vorrichtung und
Aufziehen der radioaktiven Substanz in die Spritze (10) unter Verwendung einer Abschirmungseinrichtung für den Spritzenkolben (13).

14. Verfahren nach Anspruch 13, **dadurch gekennzeichnet, daß** es das Anordnen eines medizinischen Instruments (15) oder Teils eines medizinischen Instruments (15) in der Vorrichtung und Befüllen des medizinischen Instruments mit einer radioaktiven Substanz aus der Spritze (10) umfaßt.

15. Verfahren nach einem der Ansprüche 13 oder 14, **dadurch gekennzeichnet, daß** es das Zurückführen der radioaktiven Substanz aus dem medizinischen Instrument (15) in die Spritze (10) oder in den Behälter (8) umfaßt.

## Claims

1. Apparatus for filling a medical instrument with a radioactive substance, which apparatus comprises a housing (1) screening radioactive radiation and designed to hold a syringe (10) and at least part of the medical instrument (15), a screening device (24) for an end of the syringe plunger (13) protruding from the syringe (10), and a device (23) to hold a container (8) for a radioactive substance, wherein the housing (1) comprises a cover (2) designed to cover or close the apparatus and a container (8) for a radioactive substance arranged on the housing (2), and an opening (14) for activation of the syringe plunger (13) of the syringe (10) arranged in the apparatus.

2. Apparatus according to claim 1, **characterised in that** the device (23) to hold the container (8) is designed for an arrangement and fixing of the container (8) outside the housing (1), and the housing (1) comprises an opening to create a connection with the container (8) for the supply of a radioactive substance to the housing (1).

3. Apparatus according to any of the preceding claims, **characterised in that** the housing (1) comprises a device (4, 5) which can be operated from the outside to control a valve (19, 21) arranged in the housing (1).

4. Apparatus according to any of the preceding claims, **characterised in that** it is made from a material screening radioactive radiation, in particular tungsten.

5. Apparatus according to any of the preceding claims, **characterised in that** the housing (1) comprises a window (9) and a flap (27) with which the window (9) can be closed.

6. Apparatus according to any of the preceding claims, **characterised in that** the housing (1) is designed to fix the syringe (10) and the part of the medical instrument (15) and to hold and fix one or more valves (19, 21) and connecting lines (18, 20, 22) in the housing (1).

7. Apparatus according to any of the preceding claims, **characterised in that** the housing (1) comprises an opening (16, 17) for passage of the medical instrument (15).

8. Apparatus according to any of the preceding claims, **characterised in that** it comprises a stand which is connected with the apparatus such that the apparatus and a syringe (10) arranged therein are tilted in relation to a standing surface.

9. Apparatus according to any of the preceding claims, **characterised in that** the medical instrument (15) is a catheter.

10. System for filling a container with a radioactive substance, comprising a apparatus according to any of the preceding claims, a container (8) for a radioactive substance and a syringe (10).

11. System according to claim 10, **characterised in that** it comprises a valve (19, 21) arranged in the apparatus, a medical instrument (15) arranged at least partly in the apparatus, and hose portions (18, 20, 22) to connect the syringe (10), valve (19, 21), container (8) for a radioactive substance and the medical instrument (15).

12. System according to claim 10 or 11, **characterised in that** the container (8) for a radioactive substance is removably attached to the apparatus.

13. Method for filling a radioactive substance, comprising the following steps:
insertions of a syringe (10) in an apparatus according to any of claims 1 to 10,
arrangement of a container (8) with a radioactive substance on the apparatus,
and drawing up of the radioactive substance into the syringe (10) using a screening device for the syringe plunger (13).

14. Method according to claim 13, **characterised in that** it comprises the arrangement of a medical instrument (15) or part of a medical instrument (15) in the apparatus and the filling of the medical instrument with a radioactive substance from the syringe (10).

15. Method according to one of claims 13 or 14, **characterised in that** it comprises the return of the radioactive substance from the medical instrument (15) to the syringe (10) or container (8).

## Revendications

1. Dispositif pour remplir un instrument médical avec une substance radioactive, lequel dispositif comprend un boîtier (1) aménagé pour le logement d'une seringue (10) et d'au moins une partie de l'instrument (15) médical, réfléchissant du rayonnement radioactif, un dispositif de blindage (24) pour une extrémité, dépassant d'une seringue (10), du piston de seringue (13) et un système (23) pour le logement d'un récipient (8) pour une substance radioactive, le boîtier (1) présentant un couvercle (2), qui est aménagé pour recouvrir ou fermer le dispositif et un réservoir (8) disposé sur le boîtier (2) pour une substance radioactive et présente une ouverture (14) pour l'actionnement du piston (13) de la seringue (10) disposée dans le dispositif.

2. Dispositif selon la revendication 1, **caractérisé en ce que** le système (23) pour le logement du réservoir (8) est aménagé pour un agencement et une fixation du réservoir (8) à l'extérieur du boîtier (1) et le boîtier (1) comprend une ouverture pour l'établissement d'une liaison avec le réservoir (8) pour l'arrivée d'une substance radioactive dans le boîtier (1).

3. Dispositif selon l'une des revendications précédentes, **caractérisé en ce que** le boîtier (1) comprend un système (4, 5) pouvant être actionné par l'extérieur pour la commande d'une soupape (19, 21) disposée dans le boîtier (1).

4. Dispositif selon l'une des revendications précédentes, **caractérisé en ce qu'**il est fabriqué à base d'un matériau protégeant du rayonnement radioactif, en particulier du tungstène.

5. Dispositif selon l'une des revendications précédentes, **caractérisé en ce que** le boîtier (1) comprend une fenêtre (9) et un clapet (27), avec lequel la fenêtre (9) peut être fermée.

6. Dispositif selon l'une des revendications précédentes, **caractérisé en ce que** le boîtier (1) est aménagé pour la fixation de la seringue (10) et de la partie de l'instrument (15) médical et pour le logement et la fixation d'une ou de plusieurs soupapes (19, 21) et conduites de liaison (18, 20, 22) dans le boîtier (1).

7. Dispositif selon l'une des revendications précédentes, **caractérisé en ce que** le boîtier (1) comprend une ouverture (16, 17) pour le passage de l'instrument (15) médical.

8. Dispositif selon l'une des revendications précédentes, **caractérisé en ce qu'**il comprend un support, qui est relié au dispositif, de telle sorte que le dispositif et une seringue (10) disposée à l'intérieur sont inclinés par rapport à une surface verticale.

9. Dispositif selon l'une des revendications précédentes, **caractérisé en ce que** l'instrument (15) médical est un cathéter.

10. Système pour remplir un réservoir avec une substance radioactive, qui comprend un dispositif selon l'une des revendications précédentes, un réservoir (8) pour une substance radioactive et une seringue (10).

11. Système selon la revendication 10, **caractérisé en ce qu'**il comprend une soupape (19, 21) disposée dans le dispositif, un instrument (15) médical disposé au moins partiellement dans le dispositif et des parties de flexible (18, 20, 22) pour la liaison de la seringue (10), de la soupape (19, 21), du réservoir (8) pour une substance radioactive et de l'instrument (15) médical.

12. Système selon la revendication 10 ou 11, **caractérisé en ce que** le réservoir (8) pour une substance radioactive peut être fixé de façon amovible sur le dispositif.

13. Procédé pour verser une substance radioactive, comprenant les étapes suivantes :
insertion d'une seringue (10) dans un dispositif selon l'une des revendications 1 à 10,
mise en place d'un réservoir (8) avec une substance radioactive sur le dispositif et
injection de la substance radioactive dans la seringue (10) avec utilisation d'un dispositif de blindage pour le piston de seringue (13).

14. Procédé selon la revendication 13, **caractérisé en ce qu'**il comprend la mise en place d'un instrument (15) médical ou d'une partie d'un instrument (15) médical dans le dispositif et le remplissage de l'instrument médical avec une substance radioactive provenant de la seringue (10).

15. Procédé selon l'une des revendications 13 ou 14, **caractérisé en ce qu'**il comprend le retrait de la substance radioactive de l'instrument (15) médical dans la seringue (10) ou dans le réservoir (8).
